# FASCICULE DE BREVET EUROPEEN

(11) **EP 0 754 181 B1**
(45) Date de publication et mention de la délivrance du brevet: **08.07.1998**
(21) Numéro de dépôt: 95916697.6
(22) Date de dépôt: 03.04.1995
(51) Int. Cl.: C07D 305/14, C07D 413/12

(54) **PROCEDE DE PREPARATION D'HYDROXY-7 TAXANES**
VERFAHREN ZU HERSTELLUNG VON 7-HYDROXY TAXANE
PROCESS FOR THE PREPARATION OF 7-HYDROXY TAXANES

(30) Priorité: 05.04.1994 FR 9403980
(43) Date de publication de la demande: 22.01.1997
(73) Titulaire: RHONE-POULENC RORER S.A., 92160 Antony (FR)
(72) Inventeur: BASTART, Jean-Pierre, F-77500 Lésigny (FR); BOURZAT, Jean-Dominique, F-94300 Vincennes (FR); COMMERCON, Alain, F-94400 Vitry-sur-Seine (FR); LECONTE, Jean-Pierre, F-91800 Brunoy (FR)
(74) Mandataire: Le Pennec, Magali
(86) Numéro de dépôt international: FR9500420
(87) Numéro de publication internationale: WO9526961

(56) Documents cités:
- EP-A- 0 336 840

## Description

La présente invention concerne un procédé de préparation d'hydroxy-7 taxanes de formule générale : 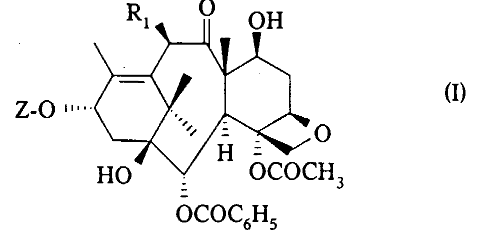 à partir d'un produit de formule générale :

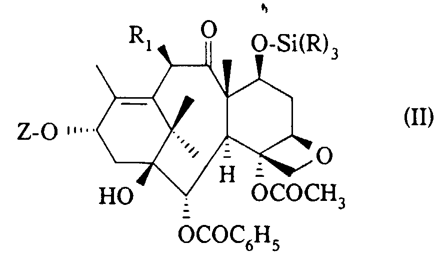

Dans les formules générales (I) et (II) :
R₁ représente un atome d'hydrogène ou un radical alcoxy contenant 1 à 4 atomes de carbone, alcoxyacétoxy dont la partie alcoyle contient 1 à 4 atomes de carbone ou alcanoyloxy contenant 1 à 4 atomes de carbone, et
Z représente un atome d'hydrogène ou un radical de formule générale : 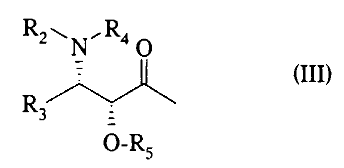 dans laquelle :
R₂ représente un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone et trifluorométhyle, ou un radical R'₂-O-CO- dans lequel R'₂ représente un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoyalmino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
   - un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
   - ou un radical hétérocyclyle azoté saturé contenant 4 à 6 atomes de carbone éventuellement substitué par un ou plusieurs radicaux alcoyles contenant 1 à 4 atomes de carbone, et
R₃ représente un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, phényle ou α- ou β-naphtyle éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, alcoylcarbamoyle, dialcoylcarbamoyle, cyano, nitro et trifluorométhyle, ou un hétérocyle aromatique ayant 5 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogènes et les radicaux alcoyles, aryles, amino, alcoylamino, dialcoylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles, et
   - ou bien R₄ représente un atome d'hydrogène et R₅ représente un atome d'hydrogène ou un groupement protecteur de la fonction hydroxy, ou bien R₄ et R₅ forment ensemble un hétérocycle saturé à 5 ou 6 chaînons.

Dans la formule générale (II), les symboles R, identiques ou différents, représentent chacun un radical alcoyle droit ou ramifié contenant 1 à 4 atomes de carbone éventuellement substitué par un radical phényle.

Plus particulièrement, la présente invention concerne un procédé de préparation des hydroxy-7 taxanes de formule générale (I) dans laquelle R₁ représente un atome d'hydrogène ou un radical alcoxy contenant 1 à 4 atomes de carbone, acyloxy contenant 1 à 4 atomes de carbone ou alcoxyacétoxy dont la partie alcoyle contient 1 à 4 atomes de carbone, Z représente un atome d'hydrogène ou un radical de formule générale (III) dans laquelle R₂ représente un radical benzoyle ou un radical R'₂-O-CO- dans lequel R'₂ représente un radical tert-butyle et R₃ représente un radical alcoyle contenant 1 à 6 atomes de carbone, alcényle contenant 2 à 6 atomes de carbone, cycloalcoyle contenant 3 à 6 atomes de carbone, phényle éventuellement substitué par un ou plusieurs atomes ou radicaux, identiques ou différents choisis parmi les atomes d'halogène (fluor, chlore) et les radicaux alcoyles (méthyle), alcoxy (méthoxy), dialcoylamino (diméthylamino), acylamino (acétylamino), alcoxycarbonylamino (tert-butoxycarbonylamino) ou trifluorométhyle ou un radical furyle-2 ou -3, thiényle-2 ou -3 ou thiazolyle-2, -4 ou -5, et, ou bien R₄ représente un atome d'hydrogène et R₅ représente un atome d'hydrogène ou un radical méthoxyméthyle, éthoxy-1 éthyle, benzyloxyméthyle ou tétrahydropyranyle, ou bien R₄ et R₅ forment ensemble un cycle oxazolidine mono-substitué ou gem-disubstitué en position-2.

Plus particulièrement encore, la présente invention concerne la préparation des hydroxy-7 taxanes de formule générale (I) dans laquelle R₁ représente un radical acétyloxy, Z représente un atome d'hydrogène ou un radical de formule générale (III) dans laquelle R₂ représente un radical benzoyle ou un radical R'₂-O-CO- dans lequel R'₂ représente un radical tert-butyle, R₃ représente un radical isobutyle, isobutényle, cyclohexyle, phényle, furyle-2, furyle-3, thiényle-2, thiényle-3, thiazolyle-2, ou thiazolyle-5 et R₄ et R₅ forment ensemble un cycle oxazolidine substitué en position 2 par un radical méthoxy-4 phényle.

Dans la demande EP 0 336 840 est décrite la préparation du taxol par déprotection simultanée d'un ester protégé sur la chaîne latérale et sur le cycle taxane au moyen d'acide chlorhydrique dans un alcool tel que l'éthanol.

Selon l'invention, les produits de formule générale (I) sont obtenus en traitant un produit de formule générale (II) par l'acide trifluoroacétique dans un solvant organique basique, tel que la pyridine éventuellement substituée par un ou plusieurs radicaux alcoyles contenant 1 à 4 atomes de carbone, éventuellement associé à un solvant organique inerte, tel que l'acétonitrile, à une température comprise entre 20 et 80°C. Il est particulièrement avantageux de mettre en oeuvre un produit de formule générale (II) dans laquelle les symboles R représentent chacun un radical éthyle.

En particulier, en opérant dans les conditions du procédé selon l'invention, le remplacement du radical trialcoylsilyloxy par un radical hydroxy s'effectue sans toucher au reste de la molécule et en particulier, lorsque Z représente un radical de formule générale (III), sans toucher au groupement protecteur représenté par R₅ ou au cycle formé par R₄ et R₅.

Les produits de formule générale (I) sont particulièrement utiles pour préparer les taxoïdes répondant aux formules générales : 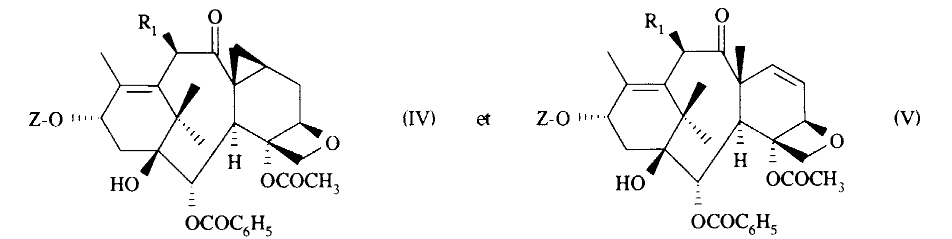 dans lesquelles R₁ et Z sont définis comme précédemment, en passant intermédiairement par un produit de formule générale : 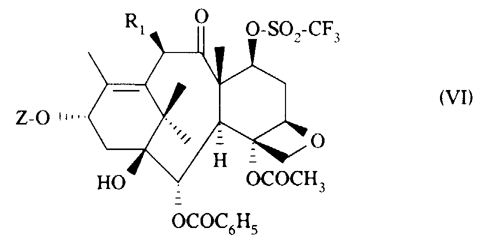 dans laquelle R₁ et Z sont définis comme précédemment.

Les produits de formule générale (IV) peuvent être obtenus par action d'un halogénure de métal alcalin (chlorure de sodium, iodure de sodium, fluorure de potassium) ou d'un azoture de métal alcalin (azoture de sodium) ou d'un sel d'ammonium quaternaire ou d'un phosphate alcalin sur un produit de formule générale (VI) dans un solvant organique choisi parmi les éthers (tétrahydrofuranne, diisopropyléther, méthyl tert-butyléther) et les nitriles (acétonitrile) pris seuls ou en mélange à une température comprise entre 20°C et la température d'ébullition du mélange réactionnel.

Les produits de formule générale (V) peuvent être obtenus en traitant un produit de formule générale (VI) par une base choisie parmi les solvants organiques basiques tels que la pyridine, les pyridines substituées par un ou plusieurs radicaux alcoyles contenant 1 à 4 atomes de carbone et la quinoléine à une température comprise entre 30 et 80°C.

Les produits de formule générale (VI) peuvent être obtenus par action d'un dérivé de l'acide trifluorométhanesulfonique tel que l'anhydride, le fluorure d'acide ou le N-phényl trifluorométhane sulfonimide en opérant dans un solvant organique inerte (hydrocarbures aliphatiques éventuellement halogénés, hydrocarbures aromatiques) en présence d'une base organique telle qu'une amine tertiaire aliphatique (triéthylamine) ou la pyridine à une température comprise entre -50 et +20°C.

Les produits de formules générales (IV) et (V) dans lesquelles Z représente un radical de formule générale (III) dans laquelle R₂ et R₃ étant définis comme précédemment, R₄ et R₅ représentent chacun un atome d'hydrogène présentent des propriétés anticancéreuses et antileucémiques remarquables.

Les exemples suivants illustrent la présente invention.

### EXEMPLE 1

A une solution de 25 g de tert-butoxycarbonyl-3 (méthoxy-4 phényl)-2 phényl- 4 oxazolidinecarboxylate-5-(2R,4S,5R) de diacétoxy-4α,10β benzoyloxy-2α époxy-5β,20 triéthylsilyloxy-7β oxo-9 hydroxy-1β taxène-11 yle-13α dans 125 cm3 d'acétonitrile et 111 cm3 de pyridine, refroidie à 5°C, on ajoute, en 45 minutes, 103,6 g d'acide trifluoroacétique. On agite pendant 15 heures à 50°C. On ajoute à nouveau 28 cm3 de pyridine et 25,9 g d'acide trifluoroacétique et agite pendant 10 heures à 50°C. On ajoute encore une fois 28 cm3 de pyridine et 25,9 g d'acide trifluoroacétique et agite pendant 15 heures à 50°C. Le mélange réactionnel est refroidi à 20°C puis est versé dans 4 litres d'eau glacée. La suspension est filtrée. Le précipité est lavé par 10 fois 200 cm3 d'eau distillée, est séché à l'air puis lavé par 140 cm3 d'oxyde d'isopropyle, essoré et enfin lavé par 2 fois 46 cm3 d'oxyde d'isopropyle. On obtient ainsi, avec un rendement de 97 %, 21,7 g de tert-butoxycarbonyl-3 (méthoxy-4 phényl)-2 phényl4 oxazolidinecarboxylate-5-(2R,4S,5R) de diacétoxy-4α,10β benzoyloxy-2α époxy-5β,20 dihydroxy-1β,7β oxo-9 taxène-11 yle-13α dont les caractéristiques sont les suivantes :
- point de fusion : 178°C
- spectre de résonance magnétique nucléaire du proton : (400 MHz ; CDCl_{3 ;} température de 323°K, δ en ppm) : 1,07 [s, 9H : C(CH₃)₃] ; 1,12 (s, 3H : CH₃) ; 1,27 (s, 3H : CH₃); 1,58 (s, 3H : CH₃); 1,66 (s, 3H : CH₃); 1,85 et 2,50 (2mt, 1H chacun : CH₂ en 6) ; 1,86 (s, 3H : COCH₃); 2,13 et 2,21 (2 dd, J = 16 et 9 Hz, 1H chacun : CH₂ en 14); 2,24 (s, 3H : COCH₃); 3,72 (d, J = 7 Hz, 1H : H en 3); 3,82 (s, 3H : OCH₃); 4,12 et 4,24 (2d, J = 8 Hz, 1H chacun : CH₂ en 20); 4,38 (dd, J = 11 et 6 Hz, 1H : H7); 4,58 (d, J = 5,5 Hz, 1H : H2'); 4,89 (dd, J = 10 et 3,5 Hz, 1H : H en 5) ; 5,43 (d, J = 5.5 Hz, 1H : H en 3'); 5,63 (d, J = 7 Hz, 1H : H en 2) ; 6,14 (t, J = 9 Hz, 1H : H en 13) ; 6,22 (s, 1H : H 10); 6,38 (s large, 1H : H 5'); 6,93 (d, J = 8.5 Hz, 2H : C₆H₅ H en ortho du OCH₃); de 7,30 à 7,50 (mt, 7H : C₆H₅ en 3' et C₆H₅ en méta du OCH₃); 7,48 (t, J = 8,5 Hz, 2H : -OCOC₆H₅ H en méta) ; 7,62 (t, J = 8,5 Hz, 1H : -OCOC₆H₅ H en para) ; 8,03 (d, J = 8,5 Hz, 2H : -OCOC₆H₅ H en ortho).

Le tert-butoxycarbonyl-3 (méthoxy-4 phényl)-2 phényl-4 oxazolidine-carboxylate-5-(2R,4S,5R) de diacétoxy-4α,10β benzoyloxy-2α époxy-5β,20 triéthylsilyloxy-7β oxo-9 hydroxy-1β taxène-11 yle-13α peut être préparé de la manière suivante :

A une solution de 147 g triéthylsilyl-7 baccatine III et de 100 g d'acide tert-butoxycarbonyl-3 (méthoxy-4 phényl)-2 phényl-4 oxazolidine-carboxylique-5 dans 720 cm3 d'acétate d'éthyle refroidie à une température voisine de 5°C, on ajoute successivement 64,7 g de dicyclohexyl-1,3 carbodiimide et 5,6 g de diméthylamino-4 pyridine.

La suspension ainsi obtenue est agitée pendant 4 heures à 20°C puis filtrée. Le filtrat est lavé par 2 fois 500 cm3 d'une solution aqueuse semi-saturée d'hydrogénocarbonate de sodium, 2 fois 500 cm3 d'eau distillée et 2 fois 500 cm3 d'une solution aqueuse saturée de chlorure de sodium.

La phase organique est séchée sur sulfate de magnésium. Après filtration et concentration à sec sous pression réduite, le produit obtenu est cristallisé dans 750cm3 de méthyl tert-butyléther, on obtient 126,9 g de tert-butoxycarbonyl-3 (méthoxy-4 phényl)-2 phényl-4 oxazolidinecarboxylate-5-(2R,4S,5R) de diacétoxy-4α,10β benzyloxy-2α époxy-5β,20 triéthylsilyloxy-7β oxo-9 hydroxy-1β taxène-11 yle-13α dont les caractéristiques sont les suivantes :
- point de fusion : 174°C
- spectre de résonance magnétique nucléaire du proton : (400 MHz ; CDCl_{3 ;} δ en ppm) : 0,58 (mt, 6H : CH₂); 0,92 (t, J = 7,5 Hz, 9H : CH₃); 1,02 (s, 3H : CH₃); 1,18 (s, 3H : CH₃); 1,68 (s, 3H : CH₃); 1,75 (s large, 1H : OH en 1); 1,87 et 2,53 (2 mt, 1H chacun : CH₂ en 6) ; 2,18 (s, 6H : CH₃ et COCH₃); 2,27 (mt, 2H : CH₂ en 14); 2,28 (s, 3H : COCH₃); 2,47 (s large, 1H : OH en 13); 3,88 (d, J = 7 Hz, 1H : H en 3); 4,13 et 4,30 (2d, J = 8,5 Hz, 1H chacun : CH₂ en 20) ; 4,50 (dd, J = 11 et 7 Hz, 1H : H7); 4,81 (mt, 1H : H en 13); 4,95 (d large, J = 10 Hz, 1H : H en 5); 5,63 (d, J = 7 Hz, 1H : H 2); 6,46 (s, 1H : H en 10); 7,46 (t, J = 8,5 Hz, 2H : -OCOC₆H₅ H en méta) ; 7,60 (t, J = 8,5 Hz, 1H : -OCOC₆H₅ H en para) ; 8,10 (d, J = 8.5 Hz, 2H : -OCOC₆H₅ H en ortho).

La triéthylsilyl-7 baccatine III peut être préparée de la manière suivante :

A une solution de 293,9 g de désacétyl-10 baccatine III dans 2,7 litres de pyridine, on ajoute, en 1 heure 20 minutes, 182 g de chlorure de triéthylsilyle. La solution obtenue est agitée pendant 40 heures à 5°C. On ajoute alors 360 g d'anhydride acétique en maintenant la température à 5°C. La suspension obtenue est agitée pendant 48 heures à 20°C puis versée sur 40 litres d'eau glacée. Le précipité obtenu est séparé par filtration puis lavé par 8 fois 2 litres d'eau et enfin dissous dans 3 litres d'acétate d'éthyle. La phase organique est séchée sur sulfate de magnésium. Après filtration et concentration sous pression réduite, le produit obtenu est cristallisé dans l'oxyde d'isopropyle. On obtient ainsi, avec un rendement de 77 %, la triéthylsilyl-7 baccatine III dont les caractéristiques sont les suivantes :
- point de fusion : 254°C
- spectre de résonance magnétique nucléaire du proton : (400 MHz ; CDCl₃, δ en ppm) : 0,58 (mt, 6H : CH₂ éthyl) ; 0,92 (t, J = 7,5 Hz, 9H : CH₃ éthyl); 1,02 (s, 3H : CH₃); 1,18 (s, 3H : CH₃); 1,68 (s, 3H : CH₃); 1,75 (s large, 1H : OH en 1) ; 1,87 et 2,53 (2 mt, 1H chacun : CH₂ en 6) ; 2,18 (s, 6H : CH₃ et COCH₃); 2,27 (mt, 2H : CH₂ en 14) ; 2,28 (s, 3H : COCH₃); 2,47 (s large, 1H : OH en 13); 3,88 (d, J = 7 Hz, 1H : H 3); 4,13 et 4,30 (2d, J = 8,5 Hz, 1H chacun : CH₂ en 20) ; 4,50 (dd, J = 11 et 7 Hz, 1H : H en 7); 4,81 (mt, 1H : H en 13); 4,95 (d large, J = 10 Hz, 1H : H en 5) ; 5,63 (d, J = 7 Hz, 1H : H 2) ; 6,46 (s, 1H: H en 10); 7,46 (t, J = 8,5 Hz, 2H : -OCOC₆H₅ H en méta) ; 7,60 (t, J = 8,5 Hz, 1H : -OCOC₆H₅ H en para) ; 8,10 (d, J = 8,5 Hz, 2H : -OCOC₆H₅ H en ortho).

### EXEMPLE 2

A une solution de 350 mg de triéthylsilyl-7 baccatine III dans 3 cm3 d'acétonitrile et 2,4 cm3 de pyridine, on ajoute 2,3 g d'acide trifluoroacétique. On agite pendant 48 heures à 50°C. Après refroidissement, le mélange réactionnel est repris par 50 cm3 de chlorure de méthylène, lavé par 2 fois 5 cm3 d'eau distillée, 10 cm3 d'acide chlorhydrique N et 2 fois 5 cm3 d'eau distillée et séché sur sulfate de magnésium. Après filtration et concentration à sec sous pression réduite, on obtient 330 mg d'un produit qui est purifié par chromatographie sur 30 g de silice contenus dans une colonne de 2 cm de diamètre en éluant par un mélange chlorure de méthylène-méthanol (99-1 en volumes). Les 300 premiers cm3 élués sont éliminés. Les 275 cm3 suivants fournissent, après concentration à sec, 235 mg de baccatine III sous forme d'une meringue blanche. Le rendement est de 83 %.

## Revendications

1. Procédé de préparation d'hydroxy-7 taxanes de formule générale : 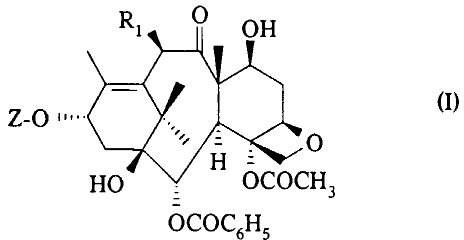 dans laquelle R₁ représente un atome d'hydrogène ou un radical alcoxy contenant 1 à 4 atomes de carbone, alcoxyacétoxy dont la partie alcoyle contient 1 à 4 atomes de carbone ou alcanoyloxy contenant 1 à 4 atomes de carbone, et
Z représente un atome d'hydrogène ou un radical de formule générale : 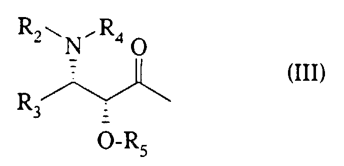 dans laquelle :
R₂ représente un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone et trifluorométhyle, ou un radical R'₂-O-CO- dans lequel R'₂ représente représente
- un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoyalmino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
- un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
- ou un radical hétérocyclyle azoté saturé contenant 4 à 6 atomes de carbone éventuellement substitué par un ou plusieurs radicaux alcoyles contenant 1 à 4 atomes de carbone, et
R₃ représente un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, phényle ou α- ou β-naphtyle éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, alcoylcarbamoyle, dialcoylcarbamoyle, cyano, nitro et trifluorométhyle, ou un hétérocyle aromatique ayant 5 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogène et les radicaux alcoyles, aryles, amino, alcoylamino, dialcoylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles, et
- ou bien R₄ représente un atome d'hydrogène et R₅ représente un atome d'hydrogène ou un groupement protecteur de la fonction hydroxy, ou bien R₄ et R₅ forment ensemble un hétérocycle saturé à 5 ou 6 chaînons, caractérisé en ce que l'on traite un un produit de formule générale : 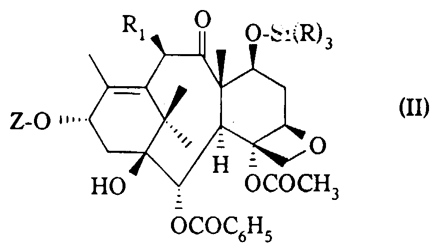 dans laquelle R₁ et Z sont définis comme précédemment et les symboles R, identiques ou différents, représentent chacun un radical alcoyle droit ou ramifié contenant 1 à 4 atomes de carbone éventuellement substitué par un radical phényle, par l'acide trifluoroacétique en opérant dans un solvant organique basique.

2. Procédé selon la revendication 1 caractérisé en ce que le solvant organique basique est choisi parmi la pyridine et les pyridines substituées par un ou plusieurs radicaux alcoyles contenant 1 à 4 atomes de carbone.

3. Procédé selon l'une des revendications 1 ou 2 caractérisé en ce que l'on opère à une température comprise entre 20 et 80°C.

4. Procédé selon l'une des revendications 1 à 3 pour la préparation d'un produit de formule générale (I) dans laquelle R₁ représente un atome d'hydrogène ou un radical alcoxy contenant 1 à 4 atomes de carbone, acyloxy contenant 1 à 4 atomes de carbone ou alcoxyacétoxy dont la partie alcoyle contient 1 à 4 atomes de carbone, Z représente un atome d'hydrogène ou un radical de formule générale (III) dans laquelle R₂ représente un radical benzoyle ou un radical R'₂-O-CO- dans lequel R'₂ représente un radical tert-butyle et R₃ représente un radical alcoyle contenant 1 à 6 atomes de carbone, alcényle contenant 2 à 6 atomes de carbone, cycloalcoyle contenant 3 à 6 atomes de carbone, phényle éventuellement substitué par un ou plusieurs atomes ou radicaux, identiques ou différents choisis parmi les atomes d'halogène (fluor, chlore) et les radicaux alcoyles (méthyle), alcoxy (méthoxy), dialcoylamino (diméthylamino), acylamino (acétylamino), alcoxycarbonylamino (tert-butoxycarbonylamino) ou trifluorométhyle ou un radical furyle-2 ou -3, thiényle-2 ou -3 ou thiazolyle-2, -4 ou -5, et, ou bien R₄ représente un atome d'hydrogène et R₅ représente un atome d'hydrogène ou un radical méthoxyméthyle, éthoxy-1 éthyle, benzyloxyméthyle ou tétrahydropyranyle, ou bien R₄ et R₅ forment ensemble un cycle oxazolidine mono-substitué ou gem-disubstitué en position-2.

5. Procédé selon l'une des revendications 1 à 3 pour la préparation d'un produit de formule générale (I) dans laquelle R₁ représente un radical acétyloxy, Z représente un atome d'hydrogène ou un radical de formule générale (III) dans laquelle R₂ représente un radical benzoyle ou un radical R'₂-O-CO- dans lequel R'₂ représente un radical tert-butyle, R₃ représente un radical isobutyle, isobutényle, cyclohexyle, phényle, furyle-2, furyle-3, thiényle-2, thiényle-3, thiazolyle-2, thiazolyle-4 ou thiazolyle-5 et R₄ et R₅ forment ensemble un cycle oxazolidine substitué en position 2 par un radical méthoxy-4 phényle.

## Patentansprüche

1. Verfahren zur Herstellung von 7-Hydroxy-taxanen der allgemeinen Formel (I) 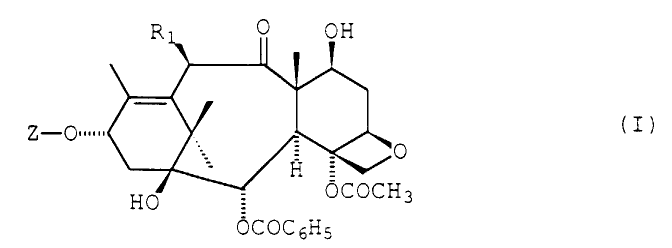 in der R₁ ein Wasserstoffatom oder einen Rest Alkoxy mit 1 bis 4 Kohlenstoffatomen, Alkoxyacetoxy, dessen Alkylteil 1 bis 4 Kohlenstoffatome enthält, oder Alkanoyloxy mit 1 bis 4 Kohlenstoffatomen darstellt, und
Z ein Wasserstoffatom oder einen Rest der allgemeinen Formel (III) 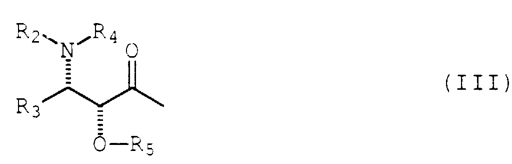 bedeutet, worin
R₂ einen Rest Benzoyl, gegebenenfalls substituiert durch ein oder mehrere, gleiche oder verschiedene Atome oder Reste, ausgewählt unter den Halogenatomen und den Resten Alkyl mit 1 bis 4 Kohlenstoffatomen, Alkoxy mit 1 bis 4 Kohlenstoffatomen und Trifluormethyl, oder einen Rest R'₂-O-CO- darstellt, worin R'₂ bedeutet
- einen Rest Alkyl mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen, Bicycloalkyl mit 7 bis 10 Kohlenstoffatomen, wobei diese Reste gegebenenfalls substituiert sind durch einen oder mehrere Substituenten, ausgewählt unter den Halogenatomen und den Resten Hydroxy, Alkoxy mit 1 bis 4 Kohlenstoffatomen, Dialkylamino, bei dem jeder Alkylteil 1 bis 4 Kohlenstoffatome enthält, Piperidino, Morpholino, 1-Piperazinyl (gegebenenfalls substituiert in -4 durch einen Rest Alkyl mit 1 bis 4 Kohlenstoffatomen oder durch einen Rest Phenylalkyl, dessen Alkylteil 1 bis 4 Kohlenstoffatome enthält), Cycloalkyl mit 3 bis 6 Kohlenstoffatomen, Cycloalkenyl mit 4 bis 6 Kohlenstoffatomen, Phenyl (gegebenenfalls substituiert durch ein oder mehrere Atome oder Reste, ausgewählt unter den Halogenatomen und den Resten Alkyl mit 1 bis 4 Kohlenstoffatomen, Alkoxy mit 1 bis 4 Kohlenstoffatomen), Cyano, Carboxy oder Alkoxycarbonyl, dessen Alkylteil 1 bis 4 Kohlenstoffatome enthält,
- einen Rest Phenyl oder α- oder β-Naphthyl, gegebenenfalls substituiert durch ein oder mehrere Atome oder Reste, ausgewählt unter den Halogenatomen und den Resten Alkyl mit 1 bis 4 Kohlenstoffatomen oder Alkoxy mit 1 bis 4 Kohlenstoffatomen, oder einen aromatischen, heterocyclischen Rest mit 5 Ringgliedern, vorzugsweise ausgewählt unter den Resten Furyl und Thienyl, oder
- den Rest eines gesättigten Stickstoff-Heterocyclus mit 4 bis 6 Kohlenstoffatomen, gegebenenfalls substituiert durch einen oder mehrere Reste Alkyl mit 1 bis 4 Kohlenstoffatomen, und
R₃ einen geraden oder verzweigten Rest Alkyl mit 1 bis 8 Kohlenstoffatomen, einen geraden oder verzweigten Rest Alkenyl mit 2 bis 8 Kohlenstoffatomen, einen geraden oder verzweigten Rest Alkinyl mit 2 bis 8 Kohlenstoffatomen, Cycloalkyl mit 3 bis 6 Kohlenstoffatomen, Phenyl oder α- oder β-Naphthyl darstellt, gegebenenfalls substituiert durch ein oder mehrere Atome oder Reste, ausgewählt unter den Halogenatomen und den Resten Alkyl, Alkenyl, Alkinyl, Aryl, Aralkyl, Alkoxy, Alkylthio, Aryloxy, Arylthio, Hydroxy, Hydroxyalkyl, Mercapto, Formyl, Acyl, Acylamino, Aroylamino, Alkoxycarbonylamino, Amino, Alkylamino, Dialkylamino, Carboxy, Alkoxycarbonyl, Carbamoyl, Alkylcarbamoyl, Dialkylcarbamoyl, Cyano, Nitro und Trifluormethyl, oder einen aromatischen Heterocyclus mit 5 Ringgliedern und einem oder mehreren gleichen oder verschiedenen Heteroatomen, ausgewählt unter den Atomen von Stickstoff, Sauerstoff oder Schwefel, bedeutet und gegebenenfalls substituiert durch einen oder mehrere, gleiche oder verschiedene Substituenten, ausgewählt unter den Halogenatomen und den Resten Alkyl, Aryl, Amino, Alkylamino, Dialkylamino, Alkoxycarbonylamino, Acyl, Arylcarbonyl, Cyano, Carboxy, Carbamoyl, Alkylcarbamoyl, Dialkylcarbamoyl oder Alkoxycarbonyl, mit der Maßgabe, daß in den Substituenten der Reste Phenyl, α- oder β-Naphthyl und der aromatischen heterocyclischen Reste die Reste Alkyl und die Teile Alkyl der anderen Reste 1 bis 4 Kohlenstoffatome enthalten, daß die Reste Alkenyl und Alkinyl 2 bis 8 Kohlenstoffatome enthalten und daß die Reste Aryl Reste Phenyl oder α- oder β-Naphthyl sind, und
- entweder R₄ ein Wasserstoffatom ist und R₅ ein Wasserstoffatom oder eine Schutzgruppe für die Hydroxyfunktion darstellt oder R₄ und R₅ zusammen einen gesättigten Heterocyclus mit 5 oder 6 Ringgliedern bilden, dadurch gekennzeichnet, daß man ein Produkt der allgemeinen Formel (II) 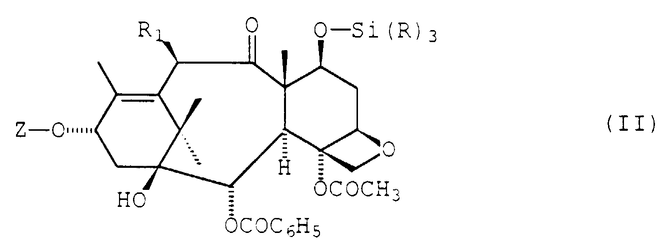 in der R₁ und Z wie vorstehend definiert sind und die Symbole R, gleich oder verschieden, jeweils einen geraden oder verzweigten Rest Alkyl mit 1 bis 4 Kohlenstoffatomen darstellen, gegebenenfalls substituiert durch einen Rest Phenyl, mit Trifluoressigsäure behandelt, wobei man in einem basischen organischen Lösungsmittel arbeitet.

2. Verfahren nach Anspruch 1, dadurch gekennzeichnet, daß das basische organische Lösungsmittel unter Pyridin und den durch einen oder mehrere Reste Alkyl mit 1 bis 4 Kohlenstoffatomen substituierten Pyridinen ausgewählt wird.

3. Verfahren nach einem der Ansprüche 1 oder 2, dadurch gekennzeichnet, daß man bei einer Temperatur zwischen 20 °C und 80 °C arbeitet.

4. Verfahren nach einem der Ansprüche 1 bis 3 zur Herstellung eines Produktes der allgemeinen Formel (I), in der R₁ ein Wasserstoffatom oder einen Rest Alkoxy mit 1 bis 4 Kohlenstoffatomen, Acyloxy mit 1 bis 4 Kohlenstoffatomen oder Alkoxyacetoxy, dessen Alkylteil 1 bis 4 Kohlenstoffatome enthält, darstellt, und Z ein Wasserstoffatom oder einen Rest der allgemeinen Formel (III) bedeutet, in der R₂ einen Rest Benzoyl oder einen Rest R'₂-O-CO- darstellt, worin R'₂ ein Rest tert.-Butyl ist und R₃ einen Rest Alkyl mit 1 bis 6 Kohlenstoffatomen, Alkenyl mit 2 bis 6 Kohlenstoffatomen, Cycloalkyl mit 3 bis 6 Kohlenstoffatomen, Phenyl, gegebenenfalls substituiert durch ein oder mehrere gleiche oder verschiedene Atome oder Reste, ausgewählt unter den Halogenatomen (Fluor, Chlor) und den Resten Alkyl (Methyl), Alkoxy (Methoxy), Dialkylamino (Dimethylamino), Acylamino (Acetylamino), Alkoxycarbonylamino (tert.-Butoxycarbonylamino) oder Trifluormethyl, oder einen Rest 2-Furyl oder 3-Furyl, 2-Thienyl oder 3-Thienyl, 2-Thiazolyl, 4-Thiazolyl oder 5-Thiazolyl darstellt, und entweder R₄ ein Wasserstoffatom ist und R₅ ein Wasserstoffatom oder einen Rest Methoxymethyl, 1-Ethoxyethyl, Benzyloxymethyl oder Tetrahydropyranyl bedeutet oder R₄ und R₅ zusammen einen in Position -2 mono-substituierten oder geminal-disubstituierten Oxazolidin-Ring bilden.

5. Verfahren nach einem der Ansprüche 1 bis 3 zur Herstellung eines Produktes der allgemeinen Formel (I), in der R₁ einen Rest Acetyloxy darstellt, Z ein Wasserstoffatom oder einen Rest der allgemeinen Formel (III) bedeutet, in der R₂ einen Rest Benzoyl oder einen Rest R'₂-O-CO- darstellt, worin R'₂ ein Rest tert.-Butyl ist, R₃ einen Rest Isobutyl, Isobutenyl, Cyclohexyl, Phenyl, 2-Furyl, 3-Furyl, 2-Thienyl, 3-Thienyl, 2-Thiazolyl, 4-Thiazolyl oder 5-Thiazolyl bedeutet und R₄ und R₅ zusammen einen in Position -2 durch einen Rest 4-Methoxy-phenyl substituierten Oxazolidin-Ring bilden.

## Claims

1. Process for the preparation of 7-hydroxytaxanes of general formula: 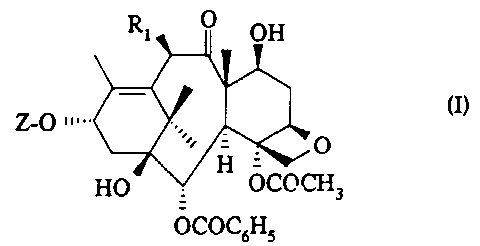 in which
R₁ represents a hydrogen atom or an alkoxy radical containing 1 to 4 carbon atoms, an alkoxyacetoxy radical in which the alkyl part contains 1 to 4 carbon atoms or an alkanoyloxy radical containing 1 to 4 carbon atoms, and
Z represents a hydrogen atom or a radical of general formula: 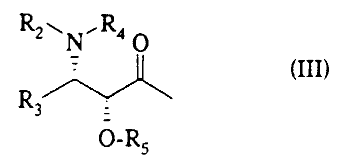 in which:
R₂ represents a benzoyl radical which is optionally substituted with one or more atoms or radicals, which may be identical or different, chosen from halogen atoms and alkyl radicals containing 1 to 4 carbon atoms, alkoxy radicals containing 1 to 4 carbon atoms and trifluoromethyl radicals, or a radical R'₂-O-CO- in which R'₂ represents
- an alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms, or a bicycloalkyl radical containing 7 to 10 carbon atoms, these radicals optionally being substituted with one or more substituents chosen from halogen atoms and hydroxyl radicals, alkoxy radicals containing 1 to 4 carbon atoms, dialkylamino radicals in which each alkyl part contains 1 to 4 carbon atoms, piperidino or morpholino radicals, 1-piperazinyl radicals (optionally substituted at -4 with an alkyl radical containing 1 to 4 carbon atoms or with a phenylalkyl radical in which the alkyl part contains 1 to 4 carbon atoms), cycloalkyl radicals containing 3 to 6 carbon atoms, cycloalkenyl radicals containing 4 to 6 carbon atoms, phenyl radicals (optionally substituted with one or more atoms or radicals chosen from halogen atoms and alkyl radicals containing 1 to 4 carbon atoms or alkoxy radicals containing 1 to 4 carbon atoms), cyano or carboxyl radicals or alkoxycarbonyl radicals in which the alkyl part contains 1 to 4 carbon atoms,
- a phenyl or α- or β-naphthyl radical which is optionally substituted with one or more atoms or radicals chosen from halogen atoms and alkyl radicals containing 1 to 4 carbon atoms or alkoxy radicals containing 1 to 4 carbon atoms or a 5-membered aromatic heterocyclic radical preferably chosen from furyl and thienyl radicals,
- or a saturated nitrogen-containing heterocyclic radical containing 4 to 6 carbon atoms which is optionally substituted with one or more alkyl radicals containing 1 to 4 carbon atoms, and
R₃ represents a straight or branched alkyl radical containing 1 to 8 carbon atoms, a straight or branched alkenyl radical containing 2 to 8 carbon atoms, a straight or branched alkynyl radical containing 2 to 8 carbon atoms, a cycloalkyl radical containing 3 to 6 carbon atoms, a phenyl or α- or β-naphthyl radical which is optionally substituted with one or more atoms or radicals chosen from halogen atoms and alkyl, alkenyl, alkynyl, aryl, aralkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro and trifluoromethyl radicals, or a 5-membered aromatic heterocycle containing one or more hetero atoms, which may be identical or different, chosen from nitrogen, oxygen or sulphur atoms and optionally substituted with one or more substituents, which may be identical or different, chosen from halogen atoms and alkyl, aryl, amino, alkylamino, dialkylamino, alkoxycarbonylamino, acyl, arylcarbonyl, cyano, carboxyl, carbamoyl, alkylcarbamoyl, dialkylcarbamoyl or alkoxycarbonyl radicals, it being understood that, in the substituents for the phenyl, α- or β-naphthyl radicals and aromatic heterocyclic radicals, the alkyl radicals and the alkyl portions of the other radicals contain 1 to 4 carbon atoms and that the alkenyl and alkynyl radicals contain 2 to 8 carbon atoms and that the aryl radicals are phenyl or α- or β-naphthyl radicals, and
- either R₄ represents a hydrogen atom and R₅ represents a hydrogen atom or a protecting group for the hydroxyl function, or R₄ and R₅ together form a 5- or 6-membered saturated heterocycle, characterized in that a product of general formula: 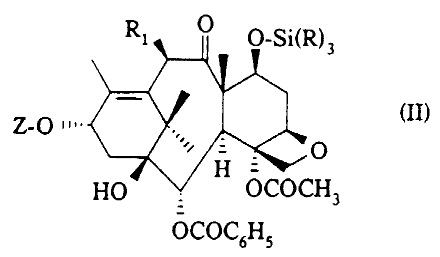 in which R₁ and Z are as defined above and the symbols R, which may be identical or different, each represent a straight or branched alkyl radical containing 1 to 4 carbon atoms which is optionally substituted with a phenyl radical, is treated with trifluoroacetic acid, working in a basic organic solvent.

2. Process according to claim 1, characterized in that the basic organic solvent is chosen from pyridine and pyridines substituted with one or more alkyl radicals containing 1 to 4 carbon atoms.

3. Process according to either of claims 1 and 2, characterized in that the process is performed at a temperature between 20 and 80°C.

4. Process according to one of claims 1 to 3 for the preparation of a product of general formula (I) in which R₁ represents a hydrogen atom or an alkoxy radical containing 1 to 4 carbon atoms, an acyloxy radical containing 1 to 4 carbon atoms or an alkoxyacetoxy radical in which the alkyl part contains 1 to 4 carbon atoms, Z represents a hydrogen atom or a radical of general formula (III) in which R₂ represents a benzoyl radical or a radical R'₂-O-CO- in which R'₂ represents a tert-butyl radical and R₃ represents an alkyl radical containing 1 to 6 carbon atoms, an alkenyl radical containing 2 to 6 carbon atoms, a cycloalkyl radical containing 3 to 6 carbon atoms, a phenyl radical which is optionally substituted with one or more atoms or radicals, which may be identical or different, chosen from halogen atoms (fluorine or chlorine) and alkyl (methyl), alkoxy (methoxy), dialkylamino (dimethylamino), acylamino (acetylamino), alkoxycarbonylamino (tert-butoxycarbonylamino) or trifluoromethyl radicals or a 2- or 3-furyl, 2- or 3-thienyl or 2-, 4- or 5-thiazolyl radical, and, either R₄ represents a hydrogen atom and R₅ represents a hydrogen atom or a methoxymethyl, 1-ethoxyethyl, benzyloxymethyl or tetrahydropyranyl radical, or R₄ and R₅ together form an oxazolidine ring which is mono-substituted or gem-disubstituted in position -2.

5. Process according to one of claims 1 to 3 for the preparation of a product of general formula (I) in which R₁ represents an acetyloxy radical, Z represents a hydrogen atom or a radical of general formula (III) in which R₂ represents a benzoyl radical or a radical R'₂-O-CO- in which R'₂ represents a tert-butyl radical, R₃ represents an isobutyl, isobutenyl, cyclohexyl, phenyl, 2-furyl, 3-furyl, 2-thienyl, 3-thienyl, 2-thiazolyl, 4-thiazolyl or 5-thiazolyl radical and R₄ and R₅ together form an oxazolidine ring which is substituted in position 2 with a 4-methoxyphenyl radical.
